Europäisches Patentamt

European Patent Office

Office européen des brevets

(19)

(11) Veröffentlichungsnummer: **0 004 945**
**B1**

(12) **EUROPÄISCHE PATENTSCHRIFT**

(45) Veröffentlichungstag der Patentschrift: **03.03.82**

(51) Int. Cl.³: **C 07 D 239/30**

(21) Anmeldenummer: **79101124.0**

(22) Anmeldetag: **12.04.79**

(54) Verfahren zur Herstellung von 2,4-Difluor-5-chlor-6-methylpyrimidin.

(30) Priorität: **22.04.78 DE 2817697**

(43) Veröffentlichungstag der Anmeldung:
**31.10.79 Patentblatt 79/22**

(45) Bekanntmachung des Hinweises auf die Patenterteilung:
**03.03.82 Patentblatt 82/9**

(84) Benannte Vertragsstaaten:
**CH DE FR GB**

(56) Entgegenhaltungen:
**DE - A - 1 670 780**
**DE - A - 1 931 640**
**DE - C - 1 044 091**
**GB 1 169 254**

(73) Patentinhaber: **BAYER AG**
**Zentralbereich Patente, Marken und Lizenzen**
**D-5090 Leverkusen 1, Bayerwerk (DE)**

(72) Erfinder: **Klauke, Erich, Dr.**
**Eichendorffweg 8**
**D-5068 Odenthal (DE)**
Erfinder: **Schündehütte, Karl Heinz**
**Klief 75**
**D-5090 Leverkusen 3 (DE)**

Courier Press, Leamington Spa, England.

# 0 004 945

## Verfahren zur Herstellung von 2,4-Difluor-5-chlor-6-methylpyrimidin

Die vorliegende Erfindung betrifft ein Verfahren zur Herstellung von 2,4-Difluor-5-chlor-6-methylpyrimidin durch Fluorierung von 2,4,5-Trichlor-6-methyl-pyrimidin.

Im allgemeinen erfolgt der Austausch aktiver Cl-Atome gegen Fluor in N-Heterocylen mit KF in polaren Lösungsmitteln wie z.B. Sulfolan, Dimethylsulfoxid, N-Methylpyrrolidon u.a. (vgl. z.B. M. Hudlicky Chemistry of Organic Fluorine Compounds 2. Aufl. J. Wiley and Sons 1976 Seite 123—4) oder in einigen Fällen auch mit NaF in Lösungsmitteln (s.o. Seite 129).

B.A. Ivin und Mitarb. beschreiben in J. General Chem. USSR Vol. 34 S. 4183 (English Translations) die Herstellung von 2,4-Difluor-6-methylpyrimidin und 2,4-Difluor-5-brom-6-methylpyrimidin durch Cl/F-Austausch mit KF.

Danach soll hierbei die Reaktion im Lösungsmittel nachteilig sein, da die Isolierung der Reaktionsprodukte "extrem schwierig" ist. Die Autoren Schlagen daher vor, die Reaktion mit KF unter Zusatz eines Katalysators im Autoklaven unter Ausschluß von Lösungsmittel durchzuführen. Die erzielte Ausbeute ist in einem Fall mit 40% d. Th. angegeben.

Als gegenüber dem erfindungsgemäßen Herstellungsverfahren nächstliegende Stand der Technik ist DE—AS 1 670 780 zu betrachten. DE—AS 1 670 780 beschreibt zwar ebenso wie die vorliegende Anmeldung eine Verfahren zur Herstellung fluorhaltiger Pyrimidine durch Umsetzung bestimmter Polyhalogenpyrimidine mit wasserfreier Fluorwasserstoffsäure unter Druck, jedoch ist das anmeldungsgemäße Verfahren im Sinne einer erfinderischen Auswahl in bezug auf DE—AS 1 670 780 aus den im folgenden genannten Gründen erfinderisch.

Sämtliche Beispiele sowie auch der allgemeine Teil der DE—AS 1 670 780 betreffen perhalogenierte Pyrimidine, die Herstellung eines Methylhalogenpyrimidin-Derivats wird weder offenbart noch nahegelegt.

In der Tat ist der Austausch des zweiten Chlorsubstituenten (in 4-Stellung des Pyrimidinringes) gegen Fluor im Falle des Ausgangsmoleküls 2,4,5-Trichlor-6-methylpyrimidin gemäß den in DE—AS 1 670 780 offenbarten Verfahrensbedingungen, d.h. bei der Umsetzung von Polyhalogenpyrimidinen mit wasserfreier HF bei 80 bis 170°C, Entspannung bei 35 bis 45 atü (d.h. deutlich über dem Partialdruck der HF) *nicht* möglich. Auf die letztgenannte Art und Weise wäre 2,4-Difluor-5-chlor-6-methylpyrimidin nicht (bzw. praktisch nicht) zugänglich.

Es wurde nun gefunden, daß man in technisch einfacher Weise durch Verwendung von wasserfreier HF als Fluorierungs- und Lösungsmittel das 2,4-Difluor-5-chlor-6-methylpyrimidin in sehr guten Ausbeuten und hoher Reinheit herstellen kann.

Zur Durchführung der Reaktion legt man im allgemeinen die wasserfreie HF in einem Autoklaven vor und tropft das 2,4,5-Trichlor-6-methylpyrimidin zu. Die Zugabe ist exotherm und man kühlt die HF zweckmäßig auf eine Temperatur von 0—10°C. Eine Cl/F-Austauschreaktion setzt bei dieser Temperatur noch nicht ein. Die HF wird zweckmäßigerweise in einem Überschuß von 5-40 Mol/Mol eingesetzt. Bevorzugt wird ein 10—20-facher Überschuß verwendet.

Anschließend wird der Autoklav verschlossen und ein Inertgas-Schutzdruck von 2—10 bar $N_2$ aufgedrückt. Dann wird aufgeheizt. Bei etwa 80° beginnt eine langsame HCL-Entwicklung, die bei 100° recht zügig verläuft. Die Reaktion wird zweckmäßig bei 100—180°C, vorzugsweise bei 120—150°C, zu Ende geführt. Der durch die Reaktion freiwerdende Chlorwasserstoff wird kontinuierlich entspannt.

Diese HCl-Entspannung ist für einen guten Umsatz und eine hohe Ausbeute an Difluorierungsprodukt von erheblicher Bedeutung. Bei den üblichen Cl/F-Austauschreaktionen mit HF wird der Entspannungsdruck so gewählt, daß er deutlich über dem Partialdruck der HF liegt. Anders ausgedrückt heißt das, daß durch die Einhaltung der Druckbedingungen der Siedepunkt der HF erheblich oberhalb der Reaktionstemperatur liegt.

Es ist nun ein wesentliches Merkmal des erfindungsgemäßen Verfahrens, daß vor allem die Einführung des zweiten Fluoratoms kurz unterhalb oder bei Rückflußbedingungen der HF durchgeführt wird.

Die Aufarbeitung eines Fluorierungsansatzes erfolgt auf einfache Weise durch Destillation.

2,4-Difluor-5-chlor-6-methylpyrimidin ist ein wichtiges Zwischenprodukt für die Herstellung von Reaktivfarbstoffen auf Basis von aminogruppenhaltigen Farbstoffen, insbesondere solchen der Azo-, Anthrachinon- und Phthalocyanin-Reihe.

Beispiel 1

In einem Rührautoklaven aus $V_4$A-Stahl mit Rückflußkühler, Manometer und einem Entspannungsventil werden 150 ml wasserfreie HF vorgelegt und bei 0—5°C 197,5 g 2,4,5-Trichlor-6-methyl-pyrimidin zugetropft. Der Autoklav wird verschlossen, 5 bar $N_2$ aufgedrückt und dann hochgeheizt. Ab 80° ist ein Druckanstieg durch die entstehende HCl zu beobachten, der bei ca. 100°C sehr zügig ist. Über das Entspannungsventil wird der Druck auf 18 bar eingestellt. Bei diesem Druck wird im Maß der HCl-Entwicklung hochgeheizt bis 165°C. Bei der gleichen Temperatur läßt man 1 1/2 Stunden nachreagieren, kühlt ab und destilliert den Ansatz auf. Man erhält nach einem Vorlauf von HF 150 g eines Grobdestillates und 11 g Destillationsrückstand. Das Destillat hat die gaschromatographische Zusammensetzung
76,5% 2,4-Difluor-5-chlor-6-methyl-pyrimidin
19,4% 2,5-Dichlor-4-fluor-6-methyl-pyrimidin
1,4% 2,4,5-Trichlor-6-methylpyrimidin
Durch Redestillation erhält man:
100 g 2,4-Difluor-5-chlor-6-methylpyrimidin als Flüssigkeit vom Kp: 158—159°C, $n_D^{20}$: 1,4778 sowie 25 g 4-Fluor-2,5-dichlor-6-methylpyrimidin mit dem Kp: 86°/20 mbar $n_D^{20}$: 1.5178.

Beispiel 2

In einem 5-1-Fluorierungsautoklaven werden 3,5 1 HF wasserfrei und 1672 g 2,4,5-Trichlor-6-methylpyrimidin unter einem Stickstoffdruck von 10 bar vorgelegt und in einer Stunde bis auf ca. 100°C geheizt.

Die in zügiger Reaktion entstehende HCl wird bei 20 bar laufend entspannt. In 1 1/2 Stunden wird auf 140°C geheizt und bei dieser Temperatur 2 Stunden belassen. Dann entspannt man den Ansatz im Verlauf von einer weiteren Stunde bis auf 17,8 bar. Dabei fällt die Innentemperatur bis auf 137°C ab. Nun wird der Ansatz abgekühlt, entspannt und durch Destillation aufgearbeitet.

Durch fraktionierte Destillation erhält man als Hauptlauf 1182 g (85% d.Th.) 2,4-Difluor-5-chlor-6-methylpyrimidin 137 g Vor- und Nachläufe sind im nächsten Ansatz wieder einsetzbar.

**Patentansprüche**

1. Verfahren zur Herstellung von 2,4-Difluor-5-chlor-6-methylpyrimidin, dadurch gekennzeichnet, daß man 2,4,5-Trichlor-6-methylpyrimidin mit Fluorwasserstoffsäure Kurz unterhalb oder bei Rückflußbedingungen der Fluorwasserstoffsäure umsetzt.

2. Verfahren gemäß Anspruch 1, dadurch gekennzeichnet, daß man 2,4,5-Trichlor-6-methylpyrimidin bei 80—180°C mit wasserfreier HF bei einem HF-Überschuß von 5—40 Mol HF pro Mol 2,4,5-Trichlor-6-methylpyrimidin kurz unterhalb oder bei Rückflußbedingungen der HF im Autoklaven umsetzt, den dabei entstehenden Chlorwasserstoff entspannt und aus dem entstehenden Reaktionsgemisch das 2,4-Difluor-5-chlor-6-methylpyrimidin destillativ abtrennt.

**Revendications**

1. Procédé de production de 2,4-difluoro-5-chloro-6-méthylpyrimidine, caractérisé en ce qu'on fait réagir de la 2,4,5-trichloro-6-méthylpyrimidine avec de l'acide fluorhydrique dans les conditions de reflux de l'acide fluorhydrique ou légèrement en dessous.

2. Procédé suivant la revendication 1, caractérisé en ce qu'on fait réagir de la 2,4,5-trichloro-6-méthylpyrimidine à 80—180°C avec HF anhydre en présence d'un excès de HF de 5—40 moles de HF par mole de 2,4,5-trichloro-6-méthylpyrimidine dans les conditions de reflux de HF en autoclave ou légèrement en dessous, on détend le gaz chlorhydrique qui est alors formé et on sépare par distillation la 2,4-difluoro-5-chloro-6-méthylpyrimidine du mélange réactionnel produit.

**Claims**

1. A process for the production of 2,4-difluoro-5-chloro-6-methyl pyrimidine, characterised in that 2,4,5-trichloro-6-methyl pyrimidine is reacted with hydrofluoric acid just below or at the reflux temperature of the hydrofluoric acid.

2. A process as claimed in Claim 1, characterised in that 2,4,5-trichloro-6-methyl pyrimidine is reacted with anhydrous hydrofluoric acid in an autoclave at 80 to 180°C just below or at the reflux temperature of the hydrofluoric acid, the hydrofluoric acid being used in an excess of from 5 to 40 moles per mole of 2,4,5-trichloro-6-methyl pyrimidine, the hydrogen chloride formed is released and the 2,4-difluoro-5-chloro-6-methyl pyrimidine is separated by distillation from the reaction mixture formed.